# EUROPEAN PATENT APPLICATION

(11) **EP 4 461 294 A1**
(43) Date of publication of application: **13.11.2024**
(21) Application number: 23172656.3
(22) Date of filing: 10.05.2023
(51) Int. Cl.: A61K 31/37, A61K 36/752, A61P 3/00, A61P 3/04, A61P 9/00, A61Q 19/00

(54) **LIPOLYTIC AND ANTIADIPOGENIC COUMARIN DERIVATIVES**

(71) Applicant: Karl-Franzens-Universität Graz, 8010 Graz (AT)
(72) Inventor: Schrammel-Gorren, Astrid, 8010 Graz (AT); Bucar, Franz, 8010 Graz (AT); Raimann, Lisa, 8010 Graz (AT)
(74) Representative: Schwarz & Partner Patentanwälte GmbH

(57) **Abstract**

The present invention relates to a composition comprising at least one compound represented by Formula (I) 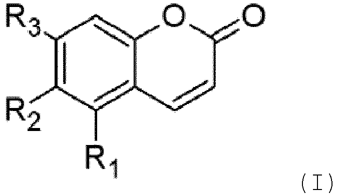 or a pharmaceutically acceptable salt thereof or a plant extract comprising said at least one compound or said salt thereof for the use in the treatment of excess bodyweight including overweight and obesity and related metabolic and cardiovascular diseases, wherein R₁ is -O-R₄, R₂ is -H or -OH and R₃ is -O-R₅, wherein R₄ and R₅ are independently an aliphatic C₁ to C₁₀ group.

## Description

### TECHNICAL FIELD

The present invention relates to the field of coumarin and coumarin derivatives used in the treatment of diseases associated with lipolysis and adipogenesis.

### BACKGROUND ART

Due to our modern lifestyle that is characterized by overnutrition, physical inactivity, and stress, prevalence of obesity and obesity-induced diseases including diabetes, cardiovascular events, and cancer has been escalating world-wide within the last decades. These non-communicable diseases impose an enormous economic and health-political burden on society and necessitates the development of novel therapeutic concepts. In this context, the interest for dietary phytochemi-cals with preventive or therapeutic effects on obesity is enormous. From the customer's perspective, natural products are often preferred since they are received without medical prescription and are believed to exhibit lower risks of adverse side effects compared to synthetic drugs.

From a metabolic point of view, obesity originates from sustained energy imbalance between energy intake and energy expenditure. A positive balance over longer periods of time due to overnutrition, physical inactivity and genetic/epigenetic factors results in storage of excess energy into triglycerides (TGs) of white adipocytes. Development of mature lipid-loaden adipocytes from precursor cells is termed adipogenesis. Vice versa, under conditions of increasing energy demand due to excessive physical activity, fasting, and disease, TGs are enzymatically cleaved into free fatty acids (FFAs) and glycerol both serving as energy substrates.

It is an object of the present invention to provide methods and means to treat excess bodyweight including overweight and obesity and related metabolic and cardiovascular diseases

### SUMMARY OF THE INVENTION

The present invention relates to a composition comprising at least one compound represented by Formula (I) 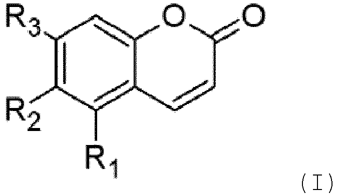 or a pharmaceutically acceptable salt thereof or a plant extract comprising said at least one compound or said salt thereof for the use in the treatment of excess bodyweight including overweight and obesity and related metabolic and cardiovascular diseases, wherein R₁ is -O-R₄, R₂ is -H or - OH and R₃ is -O-R₅, wherein R₄ and R₅ are independently an aliphatic C₁ to C₁₀ group. The composition as defined above can also be used in the treatment of a disease or a disorder associated with decreased lipolysis and/or increased adipogenesis.

It turned surprisingly out that the compounds of the present invention are able to induce lipolysis. Lipolysis is the metabolic pathway through which lipid triglycerides are hydrolyzed into glycerol and free fatty acids. This pathway is useful to mobilize stored energy within mammals from adipocytes, for instance, leading to a reduction of stored fat within these cells. The free fatty acids are typically released into the blood stream and made available for uptake to cells which require energy.

The increased lipolysis is beneficial in the treatment and/or prevention of excess bodyweight including overweight and obesity and related metabolic and cardiovascular diseases and of diseases and disorders associated with decreased lipolysis and/or increased adipogenesis, wherein adipogenesis involves the formation of adipocytes (fat cells) from stem cells.

Another aspect of the present invention relates to a method for treating excess bodyweight including overweight and obesity and related metabolic and cardiovascular diseases and/or a disease or a disorder associated with decreased lipolysis and/or increased adipogenesis and/or for inducing fat loss, resulting in weight loss, in a subject comprising the step of administering a composition as defined above to a subject in need thereof.

Yet another aspect of the present invention relates to a non-medical use of a composition as defined above for increasing lipolysis in a subject, preferably for inducing fat loss in a subject.

Excess body weight may not only have adverse effects on the health of an individual or mammal but might also have an influence of the shape of the body. Hence, the compounds and extracts of the present invention can not only be used therapeutically but also cosmetically. Due to their properties to induce lipolysis, the compounds and extracts of the present invention can be contacted with tissue containing an increased amount of adipocytes leading to a reduction of adipocytes and/or the amount of fat accumulated in these cells.

Thus, the present invention relates also to a cosmetic method for improving the texture of the skin of a subject comprising the step of administering a composition as defined herein to said subject.

### BRIEF DESCRIPTION OF THE FIGURES

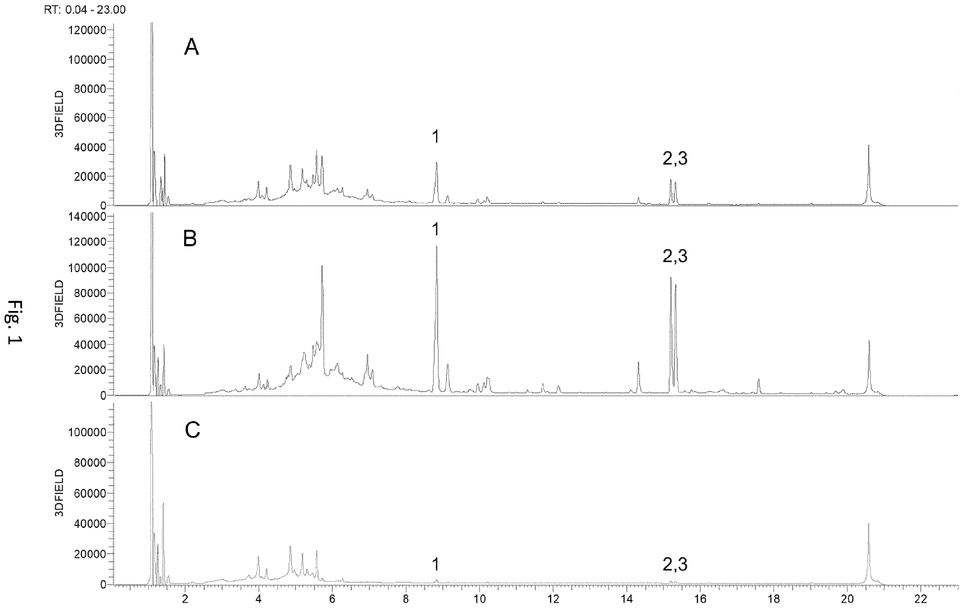

Fig. 1 shows LC-PDA chromatograms of total lemon peel (A), flavedo layer (B) and albedo layer (C). Separation on Zorbax Eclipe Plus C18 column (1.8 pm, 100 x 2.1 mm); detection at UV 318 nm; 1 = limettin; 2 = bergamottin, 3 = 5-geranyloxy-7-methoxy coumarin.

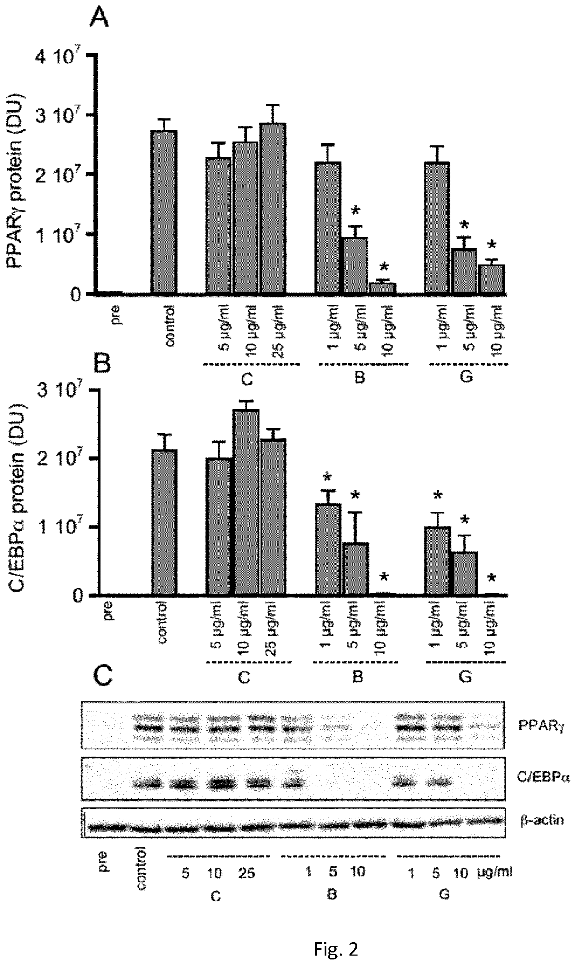

Fig. 2 shows protein expression of master regulators PPARγ (A) and C/EBPα (B) is downregulated by 5-geranyloxy-7-methoxy-coumarin and bergamottin in a concentration dependent manner. (C) Representative Western blots. Data represent mean values ± SEM of 3 individual experiments. *p<0.05 vs vehicle-treated cells (ANOVA with Dunnet's post hoc test); pre, preadipocytes; C, citropten; B, bergamottin; G, 5-geranyloxy-7-methoxycouma-rin; DU, densitometric units.

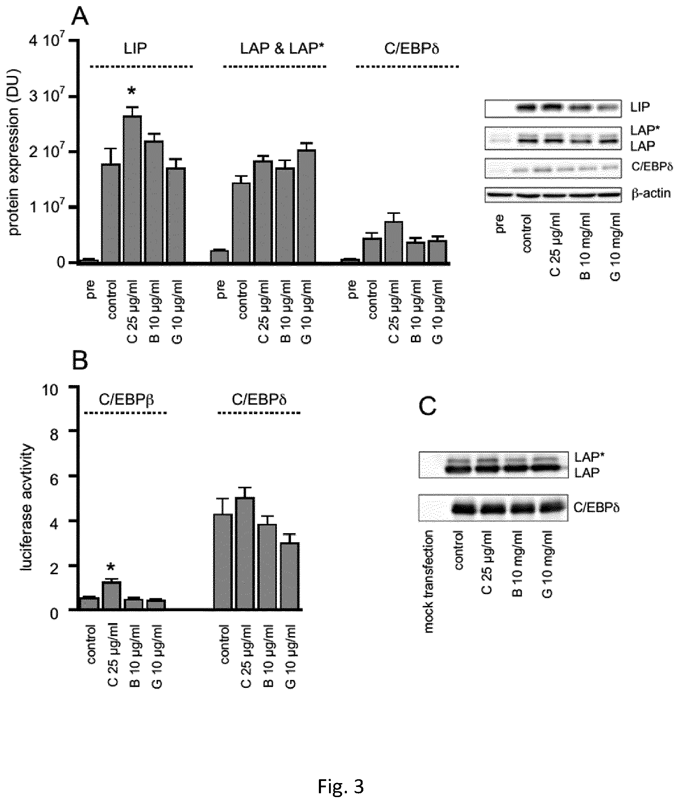

Fig. 3 shows 5-Geranyloxy-7-methoxycoumarin and bergamottin (10 ug/ml each) did not affect protein expression of C/EBPβ (isoforms LAP*, LAP, and LIP) and C/EBP6 in 3T3-L1 cells (A). Citropten (25 pg/ml) significantly upregulated expression of LIP. Data represent mean values±SEM of 3 individual experiments; *p<0.05 vs vehicle-treated cells (ANOVA) with Dunnet's post hoc test). Transcriptional activities of C/EBPβ and C/EBP6 were not affected by 5-geranyloxy-7-methoxy-coumarin and bergamottin (B). Citropten significantly upregulated activity of C/EBPβ. Data represent mean values±SEM of 4 individual experiments performed in triplicate; *p<0.05 vs mock transfection (ANOVA with Dunnet's post hoc test). (C) Assessment of transfection efficiency. C, citropten; B, bergamottin; G, 5-geranyloxy-7-methoxycoumarin; DU, densitometric units.

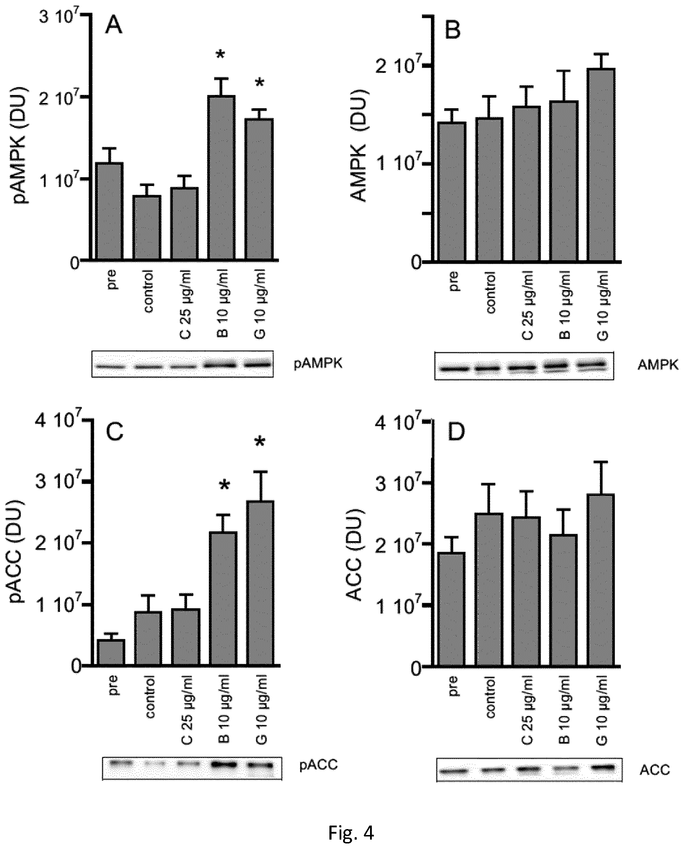

Fig. 4 shows effects of coumarins on expression and activity of AMPK and ACC in short-term experiments. Phosphorylation of (A) AMPK (Thr172) and (C) ACC (Ser79) was significantly increased by bergamottin and 5-geranyloxy-7-methoxycoumarin (10 pg/ml each). Total protein levels of AMPK (B) and ACC (D) were not affected. Data represent mean values±SEM of 7-9 experiments; *p<0.05 vs vehicle-treated cells (ANOVA with Dunnet's post hoc test) C, citropten; B, bergamottin, G, 5-geranyloxy-7-methoxycoumarin; DU, densitometric units.

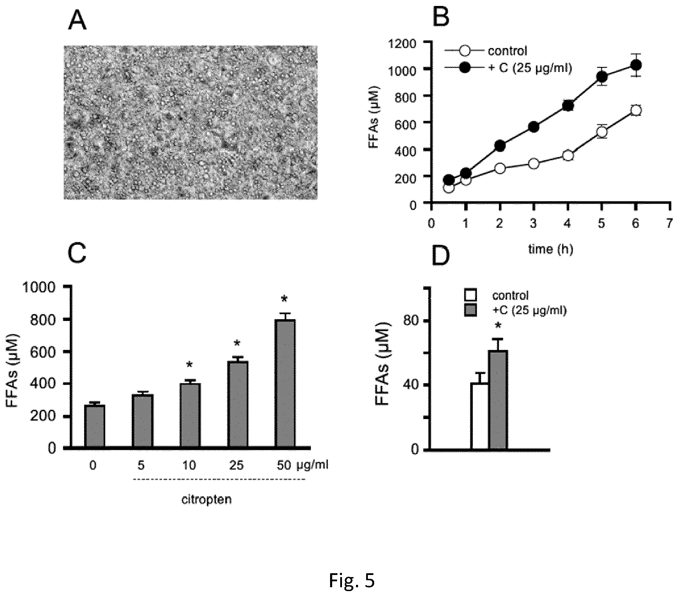

Fig. 5 shows (A) microscopic imaging of mature 3T3-L1 adipocytes (d10; 10-fold magnification). (B) Citropten (25 pg/ml) enhances forskolin-stimulated lipolysis for up to 6 h. Data represent mean values±SEM of 5-7 individual samples. (C) Concentration-dependent effect of citropten on forskolin-stimulated lipolysis (3 h). Data represent mean values±SEM of 9 individual samples. *p<0,05 versus vehicle-treated control (ANOVA with Dunnet's post hoc test). (D) Effect of citropten (25 pg/ml) on basal lipolysis (3 h). Data represent mean values±SEM of 5 experiments performed in duplicate or triplicate. *p<0,05 versus vehicle-treated control (Student's t-test for paired samples); C; citropten.

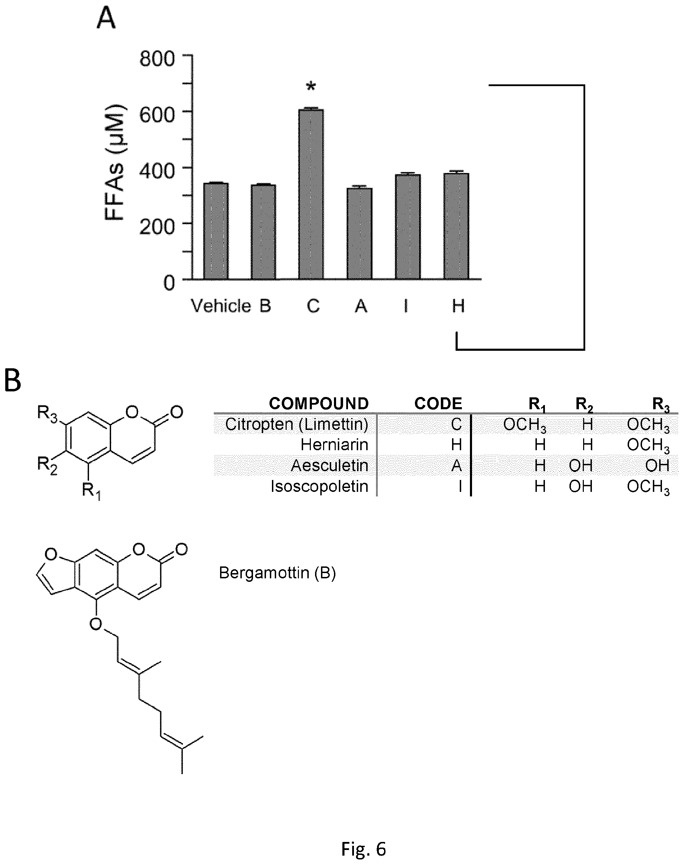

Fig. 6 shows effects of coumarin derivatives on forskolin-induced lipolysis (3h). Concentrations were 30 µM (10 µg/ml) for bergamottin (B), 120 µM (25 µg/ml) for citropten (C), 56 µM (10 µg/ml) for aesculetin (A) and isoscopoletin (I) and 141 µM (25 µg/ml) for herniarin (H), respectively. Data represent mean values±SEM of 4-10 individual samples. *p<0,05 versus vehicle-treated control (ANOVA with Dunnet's post hoc test) (B) Structures of coumarin derivatives.

### DESCRIPTION OF EMBODIMENTS

The compounds of Formula (I) of the present invention, which can also be considered as coumarin derivatives, can be synthesized chemically or can be extracted and/or isolated from natural sources. Methods for synthesizing these compounds are well known to a person skilled in the art (Ortiz-de-Elguea et al. (2021), ACS Omega, 6(44), 29483-29494).

The compounds of the present invention which are naturally occurring can be extracted and/or isolated from natural sources which typically comprise coumarin and derivatives thereof. Respective methods for extracting and/or isolating these compounds are well known to a person skilled in the art. Coumarin derivatives are exemplarily occurring in the peel of lemon fruits, preferably ripe lemon fruits (e.g. *Citrus* x *limon* L., Rutaceae). Coumarin and coumarin derivatives can be extracted using extraction methods with appropriate organic and inorganic solvents-(e.g. Stanley & Vannier (1957), J. Am. Chem. Soc., 79 (13), 3488-3491; Dugrand et al. (2013), J. Agric. Food Chem., 61, 10677-10684; Jungen et al. (2021), Food Chem., 359,129804) .

"Disease or a disorder associated with decreased lipolysis and/or increased adipogenesis", as used herein, refers to diseases and disorders which are caused or the result of a decreased lipolysis or increased adipogenesis.

The term "treatment", as used herein, refers to the medical management of a patient with the intent to cure, ameliorate, stabilize or prevent a disease or disorder. The term includes also palliative treatment, i.e., treatment designed for the relief of symptoms rather than the curing of the disease or disorder; preventative treatment, i.e., treatment directed to minimizing or partially or completely inhibiting the development of the associated disease or disorder; and supportive treatment, i.e., treatment employed to supplement another specific therapy directed toward the improvement of the associated disease or disorder. Thus, the term covers any treatment of a subject and/or mammal including the prevention of the disease from occurring in a subject and/or mammal that can be predisposed to the disease but has not yet been diagnosed as having it; the inhibition of the disease, i.e., arresting its development; and/or the relieve of the disease, i.e., causing regression of the disease.

According to a preferred embodiment of the present invention R₄ and R₅ are independently an aliphatic C₁ to C₁₀ group, preferably an aliphatic C₁ to C₆ group, more preferably an aliphatic C₁ to C₅ group, more preferably an aliphatic C₁ to C₃ group.

According to another preferred embodiment of the present invention R₄ and R₅ are independently an alkane group, an alkene group or an alkyne group.

According to a further preferred embodiment of the present invention R₄ and/or R₅ are a methyl groups.

According to a particularly preferred embodiment of the present invention the at least one compound is citropten (limettin).

Citropten is preferably used as a pure substance and more preferably as part of an extract, preferably of a plant extract, more preferably of a lemon extract. Citropten-enriched citrus extracts or citropten as a pure substance can be used for the adjuvant treatment of lipometabolic diseases, lipid metabolism disorders, obesity and secondary diseases like type 2 diabetes and cardiovascular diseases.

According to a preferred embodiment of the present invention the disease or a disorder associated with decreased lipolysis and/or increased adipogenesis is obesity.

According to another preferred embodiment of the present invention said composition comprises further coumarin or a further derivative thereof.

The composition of the present invention may comprise further active compounds, in particular coumarin or a further derivative thereof.

According to a preferred embodiment of the present invention the further coumarin derivative is selected from the group consisting of bergamottin, 5-geranyloxy-7-methoxycoumarin, herniarin, aesculetin and isoscopoletin.

5-Geranyloxy-7-methoxycoumarin-enriched citrus extracts and 5-Geranyloxy-7-methoxycoumarin as a pure substance, bergamottin-enriched citrus extracts and bergamottin as a pure substance, aesculetin-enriched extracts and aesculetin as a pure substance, and isoscopoletin-enriched extracts and isoscopoletin as a pure substance can be used for the adjuvant treatment of lipid metabolism disorders, obesity and secondary diseases (type 2 diabetes and cardiovascular diseases).

Hence, the composition may comprise a compound of Formula (I), in particular citropten (limettin), in combination with coumarin and/or one of the aforementioned coumarin derivatives. The composition comprises preferably a compound of Formula (I) and coumarin, a compound of Formula (I) and bergamottin, a compound of Formula (I) and 5-geranyloxy-7-methox-ycoumarin, a compound of Formula (I) and herniarin, a compound of Formula (I) and aesculetin, a compound of Formula (I) and isoscopoletin, a compound of Formula (I), coumarin and one or more of the aforementioned coumarin derivatives.

Particularly preferred combinations comprise limettin and 5-geranyloxy-7-methoxycoumarin-enriched citrus extracts or limettin and 5-geranyloxy-7-methoxycoumarin as pure substances and can be used for the adjuvant treatment of lipid metabolism disorders, obesity and secondary diseases (type 2 diabetes and cardiovascular diseases). Further preferred combinations comprise limettin and 5-geranyloxy-7-methoxycoumarin for the adjuvant treatment of obesity and secondary diseases (type 2 diabetes and cardiovascular diseases). Also, citrus extracts enriched with limettin, 5-geranyloxy-7-methoxycoumarin and bergamottin or limettin, 5-geranyloxy-7-methoxycoumarin and bergamottin as pure substances can be used in combination for the adjuvant treatment of lipid metabolism disorders, obesity and secondary diseases (type 2 diabetes and cardiovascular diseases) .

According to a further preferred embodiment of the present invention the composition comprises at least one excipient and/or at least one physiologically acceptable carrier.

According to another preferred embodiment of the present invention the composition is administered perorally, sublingual, percutaneously, topically, subcutaneously, rectally, vaginally, or intramuscularly to a subject in need thereof.

According to a preferred embodiment of the present invention the composition is provided in the form of an oral dosage form, a topical dosage form, a cutaneous dosage form, a transdermal dosage form, a sublingual and buccal dosage form, an intranasal dosage form, a rectal or vaginal dosage form.

According to a further preferred embodiment of the present invention the composition is provided in the form of a tablet, a capsule, a solution, an emulsion, a lotion, an ointment, a cream, a gel, a paste, a transdermal patch, a transdermal system, a cutaneous patch, a nasal spray and oromucosal solution, a spray, granules or drops, a suppositorium.

The composition of the present invention may include at least one excipient. Excipients are typically pharmacologically inactive substances used as a carrier for the active ingredients of the composition. Excipients can include substances that are used to bulk up formulations, allow for convenient and accurate dosage, stabilize the active ingredients, and make the delivery system optically and/or organoleptically acceptable. Examples of pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. Remington's Pharmaceutical Sciences, by E. W. Martin, Mack Publishing Co., Easton, PA, 19th Edition (1995), describes excipients and formulations suitable for pharmaceutical delivery of compositions. The use of pharmaceutically acceptable excipients does not imply that that product so made is useful only for pharmaceutical purposes. Rather it implies that the product is suitable for administration to or consumption by a subject or mammal, for example as a pharmaceutical or nutraceutical that is suitable for oral ingestion by a subject.

In general, the nature of the excipient will depend on the particular mode of administration being employed. For instance, solid compositions (like powder, pill, tablet or capsule forms), conventional non-toxic solid vehicles can include, for example, pharmaceutical grades of mannitol, lactose, starch, or magnesium stearate.

The composition of the present invention may be provided in a lyophilized form and is preferably adapted for the desired mode of administration, including, but not limited to tablets, gel capsules, sustained-release capsules and the like for oral administration.

Oral compositions may include an inert diluent or an edible carrier (e.g. in the form of nanoparticles or liposomes) and may be compressed into tablets or enclosed in gelatin capsules. For the purpose of oral therapeutic administration, the composition of the present invention can be incorporated with excipients and used in the form of tablets, capsules or troches. Pharmaceutically compatible binding agents and adjuvant materials can be included as part of the composition. The tablets, pills, capsules, troches and the like can contain any of the following ingredients or compounds of a similar nature: a binder such as, but not limited to, gum tragacanth, acacia, corn starch, or gelatin; an excipient such as microcrystalline cellulose, starch, or lactose; a disintegrating agent such as, but not limited to, alginic acid and corn starch; a lubricant such as, but not limited to, magnesium stearate; a glidant, such as, but not limited to, colloidal silicon dioxide; a sweetening agent such as sucrose or saccharin; and a flavoring agent such as peppermint, methyl salicylate, or fruit flavoring.

When the dosage unit form is a capsule, it can contain, in addition to material of the above type, a liquid carrier such as a fatty oil. In addition, dosage unit forms can contain various other materials, which modify the physical form of the dosage unit, for example, coatings of sugar and other enteric agents.

Capsule shells are typically made of either animal based or plant-based components that readily dissolve or disintegrate after ingestion. Animal based components may include gelatin manufactured from the collagen of animal skin and/or bone. In certain embodiments, the capsule is made of gelatin. Other suitable matrix substances such as total synthetic polymer chemicals having gelatin-like properties may be used to manufacture the capsules. Plant-based components may include vegetable starch, cellulose, hypromellose (a polymer formulated from cellulose) or pullulan (polysaccharide polymer produced from tapioca starch). In some embodiments, plant-based components may include carrageenan, potato starch, cassava starch, cornstarch, arrowroot or combinations thereof. The composition, manufacture and use of capsule shells are well known in the art.

The composition of the present invention is particularly preferred administered topically in the form of a topical dosage form, a cutaneous dosage form or a transdermal dosage form. Hence, the composition of the present invention is provided as a lotion, an ointment, a cream, a gel, a paste, a transdermal patch, a transdermal system and a cutaneous patch. The topical administration of the composition of the present invention allows, i.e., to reduce topical fat.

According to another preferred embodiment of the present invention the at least one compound represented by Formula (I) is administered to a subject in a therapeutically effective amount to treat said subject, preferably in an amount of 200 µg/kg body weight to 8000 pg/kg body weight, preferably 400 pg/kg body weight to 6000 µg/kg body weight, more preferably 500 pg/kg body weight to 5000 µg/kg body weight, even more preferably 800 µg/kg body weight to 4000 pg/kg body weight.

"Therapeutically effective amount" refers to an amount that is sufficient to achieve the desired result or to have an effect on an undesired condition. The specific therapeutically effective dose level for any particular patient will depend upon a variety of factors including the disorder being treated and the severity of the disorder; the age, body weight, general health, sex and diet of the patient; the time of administration; the route of administration; the duration of the treatment. For example, it is well within the skill of the art to start doses of a compound at levels lower than those required to achieve the desired therapeutic effect and to gradually increase the dosage until the desired effect is achieved. If desired, the effective daily dose can be divided into multiple doses for purposes of administration. Consequently, single dose compositions can contain such amounts or submultiples thereof to make up the daily dose.

According to another preferred embodiment of the present invention the at least one further coumarin or a derivative thereof is administered to a subject in an amount of 800 pg/kg body weight to 4000 pg/kg body weight.

According to a further preferred embodiment of the present invention the composition comprises 10 to 500 mg, preferably 20 to 400 mg, more preferably 30 to 300 mg, even more preferably 50 to 250 mg of said at least one further coumarin or a derivative thereof.

Another aspect of the present invention relates to a method for treating a disease or a disorder associated with decreased lipolysis and/or increased adipogenesis comprising the step of administering a composition as defined herein to a subject in need thereof.

A further aspect of the present invention relates to a non-medical use of a composition as defined herein for increasing lipolysis in a subject, preferably for inducing fat and/or topical fat loss in a subject.

Another aspect of the present invention relates to a cosmetic method for improving the texture of the skin of a subject comprising the step of administering a composition as defined herein to said subject.

According to a preferred embodiment of the present invention the composition used in the cosmetic method is topically applied to the skin to be cosmetically treated or orally applied to said subject.

According to a preferred embodiment of the present invention the composition used in the cosmetic method is applied to said subject using a patch or bandage.

### EXAMPLE

The present invention is further illustrated by the following example, however, without being restricted thereto.

In the following examples the effects of the compounds of the present invention on an established *in vitro* model is examined. 3T3-L1 cells represent a well-established and widely used *in vitro* model to study different aspects of fat cell biology (Green & Meuth (1974), Cell, 3(2), 127-133). Experimental challenge of 3T3-L1 preadipocytes with mitotic and adipogenic inducers (insulin, glucocorticoids, cAMP elevating compounds, and growth hormones) induces the adipogenic process that is characterized by a sequence of temporarily confined events driven by distinct transcription factors.

Expression and activation of CCAAT/enhancer-binding protein subtypes β and δ (C/EBPβ and C/EBPδ) dominate the early phase of adipocyte differentiation. They play a crucial role in the induction of adipogenic factors *i.e.* peroxisome proliferator activated receptor γ (PPARγ), C/EBPα, and sterol regulatory element binding protein 1 (SREBP-1) that are expressed in the intermediate phase of differentiation. PPARγ is considered as one of the key regulators of adipogenesis and most of stimulators or suppressors of adipocyte differentiation interfere with its expression and/or activity either in a direct or indirect manner. In the late phase, these transcription factors synergistically promote the terminal events of adipogenesis, *i.e.* expression of a broad range of proteins and enzymes that are required for maintenance of the mature lipid-loaded adipogenic phenotype.

Under experimental fasting conditions (deprivation lipid of energy sources and growth factors) mature lipid-enriched 3T3-L1 adipocytes are forced towards lipolysis of TGs to release FFAs and glycerol. Quantification of these products allows conclusions about overall lipolytic activity of 3T3-L1 cells and its modulation by activators or inhibitors. The lipolytic process has been studied intensively within the last decades and is well-characterized nowadays. It basically comprises three consecutive steps that are driven by three lipases termed adipose triglyceride lipase (ATGL), hormone-sensitive lipase (HSL), and monoglyceride lipase (MGL) with each enzyme catalyzing the consecutive liberation of one molecule FFA.

In the present example the effect of the compounds of the present invention, also as part of a lemon peel extract, on adipogenesis and lipolysis was examined.

### Materials and Methods

### 1.1. Materials

3T3-L1 cells (ATCC^{®} CL-173^{™}) were purchased from ATCC^{®} *via* LGC Standards GmbH (Germany). Human embryonic kidney cells 293 (HEK293) were kindly provided by Prof. Wolfgang Graier (Gott-fried Schatz Research Center for Cell Signaling, Metabolism and Aging, Medical University Graz, Austria). cOmplete Protease Inhibitor^{™} Cocktail was from Roche Life Science (Austria). Citropten (limettin; 5,7-dimethoxy-2H-chromen-2-one), bergamottin (4-[(2*E*)-3,7-dimethylocta-2,6-dienoxy]furo[3,2-g]chromen-7-one) and 5-geranyloxy-7-methoxycoumarin (5-[(2*E*)-3,7-dimethylocta-2,6-dienoxy]-7-methoxychromen-2-one) were obtained from Extrasynthese (France). Herniarin (7-methoxy-2H-chromen-2-one) was obtained from Santa Cruz Biotechnology (Germany). Isoscopoletin (6-hydroxy-7-methoxy-2H-chromen-2-one) and esculetin (6,7-dihydroxy-2H-chromen-2-one) were purchased from Carl Roth (Germany). Coumarin derivatives were dissolved in ethanol to yield 1000-fold concentrated ethanolic stock solutions. All other chemicals were purchased from Sigma, Austria.

### 1.2. Preparation of extracts and fractionation

Ripe lemon fuits (*Citrus* x *limon* L., Rutaceae) either of a commercially available brand (SanLucar^{®}, no cultivar recorded) or distinct cultivars ('Primofiore', 'Lisbon', and 'Eureka') were used for preparation of ethanolic peel extracts. Peels including flavedo and albedo layer were removed from the inner pulp by a household knife, for removing just the flavedo layer, a household peeler was used. Air-dried material was pulverized (M20 Universal mill, IKA, Germany), mixed 1:10 with 95% ethanol and extracted for 30 min at 40 °C on a shaking water bath (GFL 1086, Germany) in falcon tubes. Thereafter, extracts were centrifuged at 3100 x g (Eppendorf Centrifuge 5810R, Hamburg, Germany), supernatants collected and pellets mixed with fresh ethanol in order repeat extraction. This process was performed 3 times. Combined supernatants were concentrated to a small volume using a vacuum rotary evaporator (Büchi RE111, Flawil, Switzerland). Concentrated extracts were transferred into a snap lid glass and finally dried under nitrogen. Separated extracts from flavedo and albedo layers were prepared in the same way.

### 1.3. LC-ESI-MS analysis

Liquid chromatography coupled with mass spectrometry (LC-ESI-MS) of ethanolic lemon peel extracts was performed using a Dionex UltiMate 3000 RS System (ThermoFisher Scientific, Austria) coupled to a 3000 RS Diode Array Detector and a LTQ XL Mass Spectrometer following the method of Dugrand and coauthors (Dugrand et al., 2013, J Agric Food Chem, 61(45), 10677-10684. https://doi.org/10.1021/jf402763t). Extracts were dissolved to a concentration of 1 mg/ml and centrifuged at 2500 x g for 5 min (Eppendorf Centrifuge 5810R) at ambient temperature. Supernatants (5 µl) were injected onto an Agilent Zorbax column (Eclipse Plus C18, 1.8 pm, 100 x 2.1mm). Elution was performed at 25°C at a flow rate of 0.25 ml/min using a mobile phase gradient of formic acid in water (0.1%; A) and acetonitrile (B) and as follows: from 90% A (v/v) at 0 min to 100% B at 15 min; maintenance of 100% B until 18 min., returning to 90% A at 18.5 min, equilibration until 23 min. UV/VIS absorbance was detected in a wavelength range of 190 nm to 500 nm, chromatograms for comparison of samples were extracted at 318 nm. Mass spectral detection was carried out in m/z ranges of 50 to 2000 amu. MS conditions were set to a source voltage of 3.5 kV (positive ESI), capillary temperature of 300 °C, source temperature of 350 °C, sheath gas flow of 65 arbitrary units (arb), and auxiliary gas flow of 15 arb.

### 1.4. Cell culture

3T3-L1 preadipocytes were cultured in DMEM (D5796, high glucose) supplemented with 10% FBS, penicillin, and streptomycin at 37 °C in 5% CO₂ atmosphere and 80% humidity. For long-term experiments, cells were seeded onto 6-well plates and grown to confluence. Adipogenesis was induced 48 h *post* confluence (day 0) by addition of culture medium containing 10 pg/ml insulin, 0.4 pg/ml dexamethasone, and 500 µM 3-isobutyl-1-methylxan-thine (IBMX). Experiments were performed in the absence and presence of increasing concentrations of ethanolic citrus extracts (total peel, flavedo and albedo layers) or commercially available citropten, bergamottin and 5-geranyloxy-7-methox-ycoumarin. Ethanol (0.1%) served as vehicle. At days 3 and 5, medium was replaced by fresh medium supplemented with 10 pg/ml and 0.2 ug/ml insulin, respectively, with and without compounds. At day 7, adipogenesis was terminated by harvest of cells. In a short-term approach, differentiation was performed for 24 h. Cells were harvested by treatment with RIPA buffer (#R0278, Sigma) containing EDTA (2 mM) and cOmplete Protease Inhibitor Cocktail (total volume of 150 µl per well) and scraped off mechanically. Cell suspensions were transferred to Eppendorf vials, homogenized by repeated sonication, and kept on ice for 10 min. Aliquots were stored at 4 °C for measurement of triglycerides (TGs) and total cellular protein content. For Western blot experiments homogenates were stored at -80 °C.

### 1.5. Quantification of cellular protein and TG content

Protein concentration was determined using the Pierce^{™} BCA Protein Assay Kit from ThermoFisher Scientific according to the manufacturer's instructions. Briefly, 25 µl of samples (cells lysates) and standards (bovine serum albumin; BSA) were incubated with 200 µl reagent for 30 min at 37 °C and thereafter absorbance was measured at 562 nm using a SPECTROstar^{®} Nano microplate reader (BMG LABTECH GmbH, Germany). Cellular TGs were measured using the Infinity^{™} Triglycerides Liquid Stable Reagent according to the manufacturer's instructions using glycerol as standard. Results were expressed as nmol glycerol per mg protein.

### 1.6. Lipolysis experiments

3T3-L1 preadipocytes were seeded onto 6-well plates and cultured in DMEM (D5796; high glucose) supplemented with 10% fetal bovine serum, penicillin, and streptomycin at 37 °C in 5% CO₂ atmosphere and 80% humidity. Differentiation of cells were performed for 10 days as described in **2.4.** Mature adipocytes were washed twice in low glucose containing DMEM (D4947). Lipolysis was induced by addition of the adenylyl cyclase activator forskolin (10 µM) and cells were incubated in medium containing 2% fatty acid-free BSA for up to 6 h in the absence and presence of coumarins. In basal lipolysis experiments, forskolin was excluded. Aliquots of the medium were collected and release of free fatty acids (FFAs) was measured using the NEFA Assay Kit from FUJIFILM Wako Chemicals Europe GmbH (Neuss, Germany) (ASC-ACOD method) according to the manufacturer's instructions.

### 1.7. Western blot analysis

Lysates were denaturated by boiling with 5-fold Laemmli buffer for 10 min at 95 °C. Samples containing 15 µg of protein were separated by SDS-PAGE on 8-12% gels for 45 min at 180 V. Thereafter, proteins were transferred onto nitrocellulose membranes for 90 min (240 mA). After blocking with Tris-buffered saline containing 0.1% (v/v) Tween-20 and 5% non-fat dry milk for 1 h (ambient temperature) membranes were incubated overnight at 4 °C with primary antibodies. After incubation of membranes with respective horseradish peroxidase-conjugated IgGs immunoreactive bands were visualized using Western Bright^{™} ECL or Western Bright^{™} Quantum substrates (Biozym, Austria) and chemiluminescence was quantified with the Fusion SL Imaging System (VWR International GmbH).

### 1.8. Transcriptional activity of C/EBPβ and C/EBPδ

Transcriptional activities of C/EBPβ and C/EBP6 were measured using the C/EBP Cignal^{™} Reporter Assay (CCS-001L; dual luc) from Qiagen (Germany) and the Dual-Luciferase^{®} Reporter Assay System (E1910; Promega Corporation, Germany) as recently described (Mussbacher et al., 2019, Sci Rep, 9(1), 15403. https://doi.org/10.1038/s41598-019-51579-x). pcDNA-mC/EBPβ (Addgene plasmid # 49198) and pcDNA-mC/EBP6 (Addgene plasmid #12559) were gifts from Jed Friedman and Peter Johnson, respectively. At 80-90% confluence, HEK293 cells were co-transfected with C/EBPβ or C/EBPδ DNA (1 µg each) and Cignal Reporter (0,5 µg) in serum- and antibiotic-free medium using TurboFect^{™} Transfection Reagent (ThermoFisher Scientific). Cells were incubated for 24 h in the absence and presence of citropten, bergamottin or 5-geranyloxy-7-methoxycoumarin. Thereafter, cells were harvested and lysed according to the manufacturer's instructions. Activities of firefly luciferase and *Renilla* luciferase were sequentially measured as biolumi-nescence using the GloMax Discover System GM3000 (Promega) as described. Luminescence of each sample was normalized to *Renilla* luciferase activity.

### 1.9. Data analysis and statistics

Statistical analysis was performed using the KaleidaGraph^{®} software version 4.1.3 from Synergy software (USA). Statistical significance between two groups was analyzed using unpaired or paired t-test with equal variance. To judge for statistical significance between more than two groups, analysis of variance (ANOVA) was performed using Dunnett's *post-hoc* test. Data were expressed as mean values±standard error of the mean (SEM).

### 2. Results

### 2.1. Phytochemical analysis of lemon peel extract

Ethanolic extracts of total lemon peel as well as of separated flavedo and albedo layers were analyzed by LC-PDA-ESI-MS. Representative UV chromatograms are shown in **Fig. 1****.** In extracts of total peel and flavedo layer, the major compounds could be identified as the coumarins citropten (**1**, 8.83 min), bergamottin (**2**, 15.20 min) and 5-geranyloxy-7-methoxycoumarin (**3**, 15.32 min) by comparison of retention behavior and spectral data according to Dugrand et al. (Dugrand et al., 2013) as well as authentic reference compounds. The extracts of the albedo layer showed coumarins only in low amounts.

### 2.2. Effects of coumarins on key adipogenic transcription factors

Bergamottin and 5-geranyloxy-7-methoxycoumarin concentration-dependently suppressed protein expression of late transcription factors PPARγ (**Fig. 2A**) and C/EBPα (**Fig. 2B**) which are regarded as master regulators of the adipogenic process. By contrast, protein expression of early transcription factors C/EBPβ (isoforms LAP*, LAP and LIP) and C/EBP6 were not affected by bergamottin and 5-geranyloxy-7-methoxycoumarin at 10 pg/ml each (**Fig. 3A**). To test for potential effects of coumarins on transcriptional activities, dual luciferase reporter assays were performed in HEK293 cells. Co-transfection of cells with plasmids encoding C/EBPβ or C/EBP6 with a firefly luciferase construct under the control of a C/EBP response element was performed in the absence and presence of citropten (25 pg/ml), bergamottin (10 pg/ml) and 5-geranyloxy-7-methox-ycoumarin (10 pg/ml) for 24 h. As illustrated in **Fig. 3B****,** 5-geranyloxy-7-methoxycoumarin showed a tendency to decrease C/EBPδ-dependent transcriptional activity, however, results did not reach statistical significance. Interestingly, citropten increased both protein expression and activity of C/EBPβ. Over-expression of C/EBPβ isoforms and C/EBP6 in HEK293 cells was confirmed by Western blot (**Fig. 3C**).

### 2.3. Effects of coumarins on AMPK signaling

Recently, Ko and colleagues proposed that bergamottin exerts its antiadipogenic effect *via* activation of AMPK (Ko et al., 2018). Considering the similar inhibitory profile on adipocyte differentiation, we probed if 5-geranyloxy-7-methoxycoumarin comparably interferes with AMPK signalling. As shown in **Fig. 4A****,** phosphorylation of AMPK at Thr¹⁷² was more than doubled in the presence of 5-geranyloxy-7-methoxycoumarin (10 pg/ml) compared to controls. Moreover, phosphorylation of its downstream target acetyl-CoA carboxylase (ACC) at Ser⁷⁹ was increased about 3-fold (**Fig. 4B**). Total protein levels of these targets were not affected by coumarins within 24 h (**Figs. 4C** and 4**D**).

### 2.4. Effects of coumarins on lipolysis

Taking into account the promising results obtained for adipogenesis in 3T3-L1 cells we extended our study and investigated potential effects of lemon peel extracts and purified coumarin derivatives on lipolysis of TGs. In mature 3T3-L1 cells (**Fig. 5**), basal and forskolin-stimulated lipolysis was measured by release of free fatty acids (FFAs) into the medium. Surprisingly, presence of citropten enhanced forskolin-stimulated lipolysis in a time- and concentration-dependent manner. Thus, in the presence of 25 pg/ml (≈ 120 µM) citropten, release of FFAs was significantly increased within 0.5 and 6 h (**Fig. 5B**). Since the effect of citropten was maximal at 3-4 h after induction of lipolysis (185-190% of controls) a time span of 3 h was chosen for further experiments. In **Fig. 5C****,** the effect of increasing citropten concentrations on forskolin-induced lipolysis is shown. A significant effect was already observed at 10 pg/ml (≈ 48 µM) of the coumarin. Besides the effect on stimulated lipolysis, basal release of FFAs was also increased in the presence of citropten (**Fig**. **5D**). The pro-lipolytic effect was found selective for citropten since structural related coumarin derivatives such as bergamottin, esculetin, herniarin, and isoscopoletin did not significantly enhance lipolysis (**Fig. 6A**). The structures of these compounds areare depicted in **Fig. 6B****.**

## Claims

1. A composition comprising at least one compound represented by Formula (I) 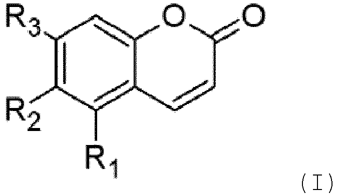 or a pharmaceutically acceptable salt thereof or a plant extract comprising said at least one compound or said salt thereof for the use in the treatment of excess bodyweight including overweight and obesity and related metabolic and cardiovascular diseases, wherein R₁ is -O-R₄, R₂ is -H or -OH and R₃ is -O-R₅, wherein R₄ and R₅ are independently an aliphatic C₁ to C₁₀ group.

2. The composition for the use according to claim 1, wherein R₄ and R₅ are independently an aliphatic C₁ to C₁₀ group, preferably an aliphatic C₁ to C₆ group, more preferably an aliphatic C₁ to C₅ group, more preferably an aliphatic C₁ to C₃ group.

3. The composition for the use according to claim 1 or 2, wherein R₄ and R₅ are independently an alkane group, an alkene group or an alkyne group.

4. The composition for the use according to any one of claims 1 to 3, wherein R₄ and/or R₅ are a methyl group.

5. The composition for the use according to any one of claims 1 to 4, wherein the at least one compound is citropten.

6. The composition for the use according to any one of claims 1 to 5, wherein said composition comprises further coumarin or a further derivative thereof, wherein the further coumarin derivative is preferably selected from the group consisting of bergamottin, 5-geranyloxy-7-methoxycoumarin, herniarin, aesculetin and isoscopoletin.

7. The composition for the use according to any one of claims 1 to 6, wherein the composition comprises at least one excipient and/or at least one physiologically acceptable carrier.

8. The composition for the use according to any one of claims 1 to 7, wherein the composition is administered perorally, sublingual, percutaneously, topically, subcutaneously, rectally, vaginally, or intramuscularly to a subject in need thereof.

9. The composition for the use according to any one of claims 1 to 8, wherein the at least one compound represented by Formula (I) is administered to a subject in an amount of 200 µg/kg body weight to 8000 pg/kg body weight, preferably 400 pg/kg body weight to 6000 µg/kg body weight, more preferably 500 pg/kg body weight to 5000 µg/kg body weight, even more preferably 800 µg/kg body weight to 4000 pg/kg body weight.

10. The composition for the use according to any one of claims 1 to 9, wherein the composition comprises 10 to 500 mg, preferably 20 to 400 mg, more preferably 30 to 300 mg, even more preferably 50 to 250 mg of said at least one compound represented by Formula (I).

11. The composition for the use according to any one of claims 6 to 10, wherein the at least one further coumarin or a derivative thereof is administered to a subject in an amount of 800 pg/kg body weight to 4000 pg/kg body weight.

12. The composition for the use according to any one of claims 6 to 11, wherein the composition comprises 10 to 500 mg, preferably 20 to 400 mg, more preferably 30 to 300 mg, even more preferably 50 to 250 mg of said at least one further coumarin or a derivative thereof.

13. Non-medical use of a composition as defined in any one of claims 1 to 12 for increasing lipolysis in a subject, preferably for inducing fat loss in a subject.

14. A cosmetic method for improving the texture of the skin of a subject comprising the step of administering a composition as defined in any one of claims 1 to 12 to said subject.

15. Cosmetic method according to claim 14, wherein the composition is topically applied to the skin to be cosmetically treated or orally applied to said subject.
